**Europäisches Patentamt**

**European Patent Office**

**Office européen des brevets**

(19)

(11) Veröffentlichungsnummer: **0 123 778**
**A2**

# EUROPÄISCHE PATENTANMELDUNG

(12)

(21) Anmeldenummer: **84100270.2**

(22) Anmeldetag: **12.01.84**

(51) Int. Cl.³: **A 61 B 3/02**

(30) Priorität: **02.05.83 DE 3315939**

(43) Veröffentlichungstag der Anmeldung: **07.11.84**
Patentblatt 84/45

(84) Benannte Vertragsstaaten: **DE FR GB**

(71) Anmelder: **Oculus Optikgeräte GmbH,
D-6331 Dutenhofen (DE)**

(72) Erfinder: **Kirchhübel, Rainer, Dipl.-Ing.,
Schwalbengraben 62c, D-6330 Wetzlar-Dalheim (DE)**
Erfinder: **Veit, Wolfgang, Am Schiessplatz 6,
D-6336 Solms-Burgsolms (DE)**

(74) Vertreter: **Missling, Arne, Dipl.-Ing. et al, Patentanwälte
Dipl.-Ing. R. Schlee Dipl.-Ing. A. Missling
Bismarckstrasse 43, D-6300 Giessen (DE)**

(54) **Vorrichtung zur Augenrefraktionsbestimmung.**

(57) Vorrichtungen für die Augenrefraktionsbestimmungen haben einen in Blickrichtung des Probanden (2) angeordneten Teilerspiegel (3), der im bildseitigen Strahlengang einer ersten Abbildungslinse angeordnet ist. Der Brennpunkt der zweiten Abbildungslinse (9) fällt mit dem Brennpunkt der ersten Abbildungslinse (6) zusammen. Nahe der zweiten Abbildungslinse (9) ist im Strahlengang ein Phoropter (12) sowie ein Kollimator (18) angeordnet. Des weiteren dienen zwei Umlenkspiegel (10, 11) zur Umlenkung des Strahlenganges. Damit diese Vorrichtung sehr kompakt gebaut werden kann und der Arzt bei Bestätigung des Phoropters seitlich neben dem Patienten sitzen kann und die Darstellung der Optotypen seiten- und achsenrichtig ist, ist der Phorpter (12) seitlich und in etwa parallel zur Blickrichtung des Probanden angeordnet, wobei in dem Strahlengang (7) zwischen dem Phoropter (12) und dem vor dem Probanden angeordneten Teilerspiegel (3) zwei Umlenkspiegel angeordnet sind, deren Abstand und Winkelstellung zueinander so bemessen sind, daß die Strahlenlänge für jedes Auge gleichlang bleibt. In den Strahlengang der ersten Abbildungslinse (6) und den vor dem Probanden liegenden Teilerspiegel (3) ist ein weiterer Umlenkspiegel (4) eingeschaltet.

OCULUS Optikgeräte GmbH,
6331 Dutenhofen


Vorrichtung zur Augenrefraktionsbestimmung


Die Erfindung betrifft eine Vorrichtung zur Augenrefraktionsbestimmung mit einem in Blickrichtung des Probanden angeordneten Teilerspiegel, der im bildseitigen Strahlengang einer ersten Abbildungslinse angeordnet ist, mit einer zweiten Abbildungslinse, deren Brennpunkt mit der ersten Abbildungslinse zusammenfällt, mit einem nahe der zweiten Abbildungslinse liegenden Phoropter, mit einem Kollimator, z.B. in Form eines Hohlspiegels, der im Strahlengang vor dem Phoropter angeordnet ist und in dessen Brennebene über einen Teilerspiegel eingespiegelte Optotypen angeordnet sind und mit zwei Umlenkspiegeln im Strahlengang, von denen einer vor dem Phoropter und der andere unterhalb des in Blickrichtung des Probanden liegenden Teilerspiegels angeordnet ist.

Eine Vorrichtung zur Augenrefraktionsbestimmung der vorstehend genannten Art ist aus der DE-OS 28 15 120 bekannt. Der Vorteil einer derartigen Vorrichtung besteht darin, daß der Proband die Sehprobe ohne die Verwendung einer Probierbrille durchführen kann, wobei gleichzeitig sein Gesichtsfeld nicht eingeschränkt wird. Die Linse des Phoropters wird je nach Wunsch entweder in der Hauptebene des zu untersuchenden Auges abgebildet oder aber in einem dem Scheitelabstand eines Brillenglases entsprechenden Abstand vor dem Auge. Hiermit können die gleichen Wirkungen erzielt werden, als würde der Proband durch ein in einer Probierbrille oder in einem vor dem Auge befindlichen Phoropter befindliches Probierglas schauen. Der hinter dem Phoropter angeordnete Kollimator, der vorteilhaft aus einem Hohlspiegel besteht, bildet die in seiner Brennebene angeordneten Optotypen über einen halbdurchlässigen Spiegel, die beiden Abbildelinsen, im Unendlichen ab, so daß der Proband die Optotypen über den vor seinem Auge befindlichen halbdurchlässigen Spiegel im freien Raum wahrnimmt.

Eine vorstehende Vorrichtung zur Augenrefraktionsbestimmung weist somit wesentliche Vorteile gegenüber den bekannten Vorrichtungen auf, zumal bei dieser Vorrichtung ein handelsüblicher Phoropter verwendbar ist. Allerdings ist diese bekannte Vorrichtung in baulicher Hinsicht nicht befriedigend, denn der Phoropter, der zwar unterhalb der Blickrichtung des Probanden angeordnet ist, befindet sich unmittelbar vor dem Patienten, so daß auch der Arzt beim Einstellen vor dem Patienten steht. Dies ist insofern nachteilig, da der Arzt jedes Mal seinen Standort wechseln muß. Ein weiterer Nachteil dieses bekannten Gerätes besteht darin, daß die Baulänge der Vorrichtung relativ groß ist, so daß diese einen nicht unerheblichen Platzbedarf erfordert. Darüberhinaus zeigt die bekannte Vorrichtung den Nachteil, daß die Optotypen nicht achsenrichtig dargestellt sind.

Der Erfindung liegt die Aufgabe zugrunde, eine Vorrichtung zur Augenrefraktionsbestimmung der eingangs genannten Art so auszubilden, daß die Größe der Vorrichtung wesentlich kleiner gebaut werden kann, der Arzt bei der Bedienung des Phoropters seitlich neben dem Patienten sitzen kann und die Darstellung der Optotypen seiten- und achsenrichtig ist.

Diese Aufgabe wird erfindungsgemäß mit den Merkmalen des kennzeichnenden Teils des Anspruches 1 gelöst.

Bei einer erfindungsgemäß ausgebildeten Vorrichtung ist der Phoropter parallel zur Blickrichtung des Patienten, jedoch seitlich hierzu versetzt, so daß die Blickrichtungen von Proband und Arzt sich kreuzen. Der Arzt kann somit den Phoropter, der handelsüblich ausgebildet ist, bedienen, ohne daß die Blickrichtung des Probanden gestört wird. Der Phoropter kann entweder aufrecht stehend oder liegend angeordnet sein. Vorteilhaft befinden sich die Optotypen auf der anderen Seite der Blickrichtung des Probanden und werden quer zu dieser über den Kollimator, z.B. einen Hohlspiegel, in den Phoropter eingespiegelt. Damit bei der Queranordnung des Phoropters ein seiten- wie achsenrichtiges Bild erhalten wird, sind in den Strahlengang im Anschluß an die zweiten Abbildungslinsen zwei Umlenkspiegel eingeschaltet, die derart zueinander angeordnet sind, daß durch die Umlenkung mittels dieser beiden Spiegel die Strahlenlängen für das linke und das rechte Auge nicht verändert werden. Durch diese beiden Umlenkspiegel, die den zweiten Abbildungslinsen nachgeschaltet sind, wird ein seitenrichtiges Bild erzeugt, d.h. die Einstellungen des Phoropters durch den Arzt erfolgen in der bisher üblichen Art und Weise. Nach der ersten Abbildungslinse ist ein weiterer Umlenkspiegel zwischengeschaltet, wodurch ein achsenrichtiges Bild erhalten wird, d.h. wenn am Phoropter ein Zylinder mit z.B. 135° eingestellt wird, wird dieser auch entsprechend achsenrichtig in das Auge des Patienten eingespiegelt, so daß diesbezügliche Bedienungsfehler ausgeschaltet sind.

Weitere vorteilhafte Ausgestaltungen der Erfindung gehen aus den Unteransprüchen in Verbindung mit Beschreibung und Zeichnung hervor.

Zwei Ausführungsbeispiele der Erfindung sind im folgenden anhand der Zeichnung näher beschrieben, in dieser zeigen:

Fig. 1  ein Ausführungsbeispiel einer Vorrichtung zur Augenrefraktionsbestimmung mit einem stehend angeordneten Phoropter,

Fig. 2  einen Schnitt nach Linie II-II in Fig. 1,

Fig. 3  einen Schnitt nach Linie III-III in Fig. 1,

Fig. 4  ein weiteres Ausführungsbeispiel einer Vorrichtung zur Augenrefraktionsbestimmung mit einem liegend angeordneten Phoropter und

Fig. 5  einen Schnitt nach Linie V-V in Fig. 4.

Von dem nicht weiter dargestellten Probanden sind die beiden Augen mit 1 und 2 bezeichnet. Der Proband blickt durch einen Teilerspiegel 3 , in den von unten her über einen Umlenkspiegel 8 die Optotypen 5 eingespiegelt werden.

Den ersten Abbildungslinsen 6, die zusammen mit den zweiten Abbildungslinsen 9 ein Fernrohr bilden, sind zwei Umlenkspiegel 10 und 11 nachgeschaltet. Diese beiden Umlenkspiegel 10, 11 sind derart zueinander und in einem solchen Winkel angeordnet, daß die Laufstrecke des Lichtstrahles von der zweiten Abbildungslinse 9 zur jeweils zugehörigen ersten Abbildungslinse 6 gleich ist. Des weiteren wird durch diese beiden Umlenkspiegel 10 und 11 eine seitenrichtige Vertauschung der jeweiligen Lichtstrahlen erhalten, so daß die entsprechende Linse des Phoropters dem entsprechenden Auge des Patienten, so wie es bisher üblich war, zugeordnet ist.

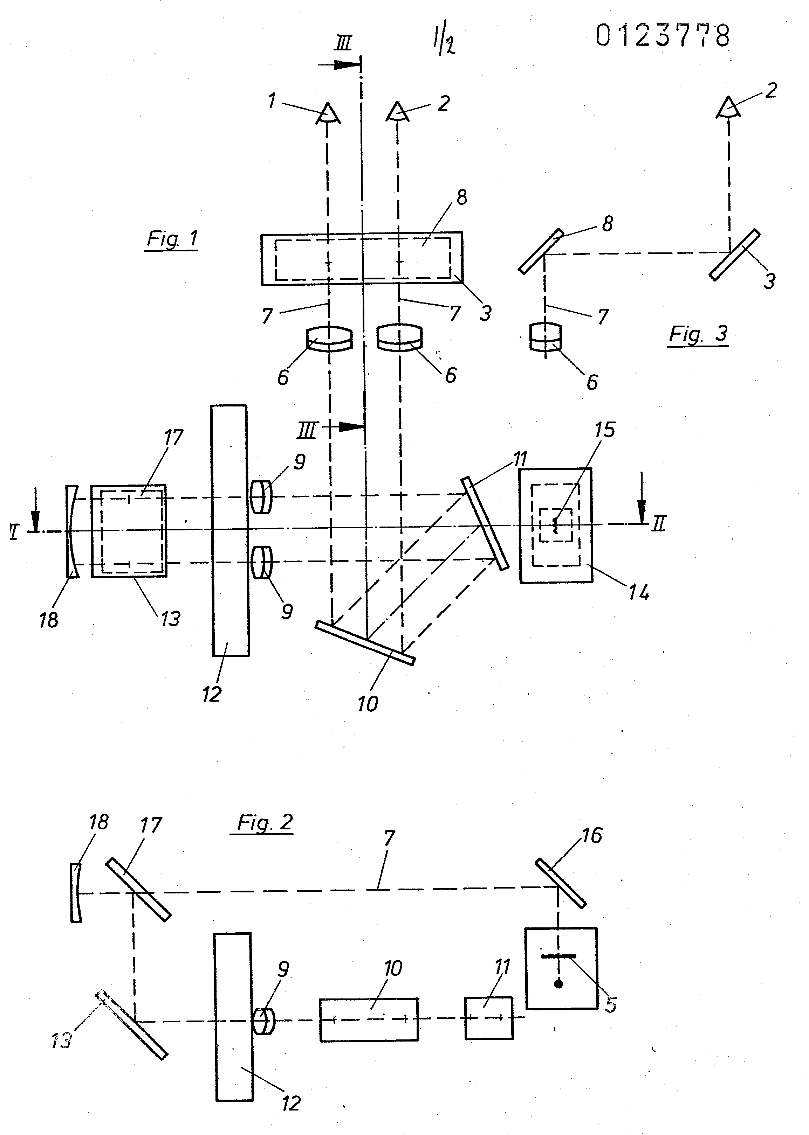

In unmittelbarer Nähe der zweiten Abbildungslinse 9 ist der Phoropter 12 angeordnet, dem die Optotypen 5 über einen Umlenkspiegel 13 eingespiegelt werden. Die Optotypen 5 werden mittels eines Projektors 14 erzeugt, der mit einer Halogenlampe 15 ausgestattet ist. Der Probjektor ist in Fig. 1 eingezeichnet, während er in Fig. 2 der Übersichtlichkeit halber weggelassen worden ist. Der Projektor 14 projeziert die Optotypen nach unten auf einen Umlenkspiegel 16    , von dem aus der Strahl 7 auf einen zweiten Teilerspiegel 17 projeziert wird. Hinter dem Teilerspiegel ist ein Hohlspiegel 18 angeordnet, in dessen Brennebene die Optotypen 5 angeordnet sind. Über den Teilerspiegel 17 werden die Optotypen in eine darüberliegende Ebene auf den Umlenkspiegel 13 projeziert und von dort zum Phoropter 12. Durch die seitliche Anordnung des Phoropters 12 sowie des Projektors 14 zur Blickrichtung des Probanden kann das Gerät sehr kurz gebaut werden, so daß es nur einen geringen Platzbedarf erfordert. Da des weiteren der Phoropter 12 um 90° zum Patienten versetzt angeordnet ist, sitzt der Arzt neben dem Patienten und somit außerhalb dessen Blickrichtung, so daß die Bedienung des Phoropters ohne einen notwendigen Platzwechsel des Arztes erfolgen kann. Da darüberhinaus durch die beiden Umlenkspiegel 10 und 11 und den Umlenkspiegel 4 dafür Sorge getragen ist, daß auch bei parallel zur

- 6 -

0123778

Blickrichtung des Probanden angeordnetem Phoropter eine seiten- und achsenrichtige Einspiegelung in die Patientenaugen erfolgt, ist auch eine Umgewöhnung des Arztes bei der Bedienung oder gar eine Umskalierung des Phoropters nicht erforderlich.

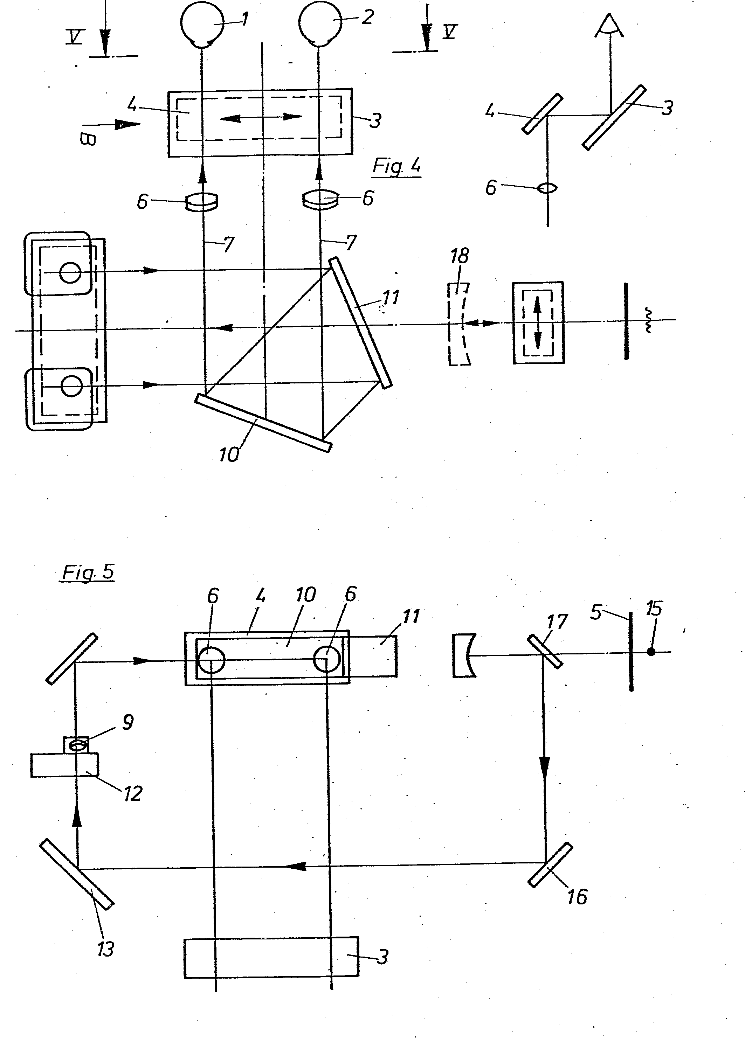

Das in den Fig. 4 und 5 dargestellte Ausführungsbeispiel einer Vorrichtung zur Augenrefraktionsbestimmung unterscheidet sich von dem in den Fig. 1 bis 3 beschriebenen im wesentlichen dadurch, daß der Phoropter im Gegensatz zu dem Ausführungsbeispiel nach den Fig. 1 bis 3 liegend seitlich der Blickrichtung des Probanden angeordnet ist. Man erhält hier gleichermaßen eine kurzbauende Vorrichtung, jedoch ist diese Vorrichtung etwas breiter bauend als eine solche nach den Fig. 1 bis 3. Gleiche Teile sind mit gleichen Bezugszeichen versehen. Die Optotypen 5 liegen wiederum im Brennpunkt eines Hohlspiegels 18, zwischen dem und den Optotypen ein Teilerspiegel 17 angeordnet ist. Von diesem Teilerspiegel gelangt der Lichtstrahl auf zwei Umlenkspiegel 16 und 13, die in einer Ebene oberhalb des Teilerspiegels 17 liegen. Der Phoropter 12 ist in einer Ebene angeordnet, die zwischen der Ebene der Umlenkspiegel 13 und 16 sowie des Teilerspiegels 17 liegt. In unmittelbarem Anschluß an den Phoropter 12 ist die zweite Abbildungslinse 9 angeordnet. Direkt unterhalb des Phoropters ist ein weiterer Umlenkspiegel 10 angeordnet, der mit einem Umlenkspiegel 11 zusammenwirkt, die beide wiederum so angeordnet sind, daß die Strahlenlänge für beide Augen gleich ist. Die Strahlen gelangen dann, wie auch beim Ausführungsbeispiel nach Fig. 1, in die ersten Abbildungslinsen 6 und von dort aus zu dem Umlenkspiegel 8

der die Strahlen auf den Teilerspiegel 3 wirft. Hinter diesem Teilerspiegel 3 befinden sich dann die Augen 1, 2 des Probanden.

Ansprüche:

1. Vorrichtung zur Augenrefraktionsbestimmung mit einem in Blickrichtung des Probanden angeordneten Teilerspiegel, der im bildseitigen Strahlengang einer ersten Abbildungslinse angeordnet ist, mit einer zweiten Abbildungslinse, deren Brennpunkt mit der ersten Abbildungslinse zusammenfällt, mit einem nahe der zweiten Abbildungslinse liegenden Phoropter, mit einem Kollimator, z.B.in Form eines Hohlspiegels, der im Strahlengang vor dem Phoropter angeordnet ist und in dessen Brennebene über einen Teilerspiegel eingespiegelte Optotypen angeordnet sind und mit zwei Umlenkspiegeln im Strahlengang, von denen einer vor dem Phoropter und der andere unterhalb des in Blickrichtung des Probanden liegenden Teilerspiegels angeordnet ist, dadurch gekennzeichnet, daß der Phoropter (12) seitlich und in etwa parallel zur Blickrichtung des Probanden angeordnet ist, daß in den Strahlengang (7) zwischen dem Phoropter (12) und dem vor dem Probanden angeordneten Teilerspiegel (3) zwei Umlenkspiegel (10, 11) angeordnet sind, deren Abstand und Winkelstellung zueinander so bemessen sind, daß die Strahlenlänge für jedes Auge gleich lang bleibt.

2. Vorrichtung nach Anspruch 1, dadurch gekennzeichnet, daß die Optotypen (5) auf der dem Phoropter (12) gegenüberliegenden Seite relativ zur Blickrichtung des Probanden angeordnet sind.

3. Vorrichtung nach Anspruch 2, dadurch gekennzeichnet, daß die Optotypen (5) dem Phoropter (12) gegenüberliegend angeordnet sind und der von den Optotypen (5) kommende Strahlengang (7) senkrecht zur und unterhalb der Blickrichtung des Probanden verläuft.

4. Vorrichtung nach Anspruch 1 bis 3, dadurch gekennzeichnet, daß die erste (6) und die zweite Abbildungslinse (9) ein Fernrohr mit einem Abbildungsmaßstab von 1 : 1 bilden.

5. Vorrichtung nach einem der Ansprüche 1 bis 4, dadurch gekennzeichnet, daß der Phoropter (12) horizontal liegend angeordnet ist.

0123778

Fig. 1

Fig. 2

Fig. 3

0123778

Fig. 4

Fig. 5